# EUROPÄISCHE PATENTSCHRIFT

(11) **EP 1 513 533 B1**
(45) Veröffentlichungstag und Bekanntmachung des Hinweises auf die Patenterteilung: **17.03.2010**
(21) Anmeldenummer: 03755107.4
(22) Anmeldetag: 21.05.2003
(51) Int. Cl.: A61K 31/496, A61K 31/135, A61K 31/60, A61K 31/495, A61P 25/16

(54) **PHARMAZEUTISCHE WIRKSTOFFKOMBINATION SOWIE DEREN VERWENDUNG**
PHARMACEUTICAL ACTIVE SUBSTANCE COMBINATION AND THE USE THEREOF
COMBINAISON D'AGENTS ACTIFS PHARMACEUTIQUES ET UTILISATION

(30) Priorität: 24.05.2002 DE 10223254; 25.11.2002 DE 10254822; 07.03.2003 DE 10310396
(43) Veröffentlichungstag der Anmeldung: 16.03.2005
(73) Patentinhaber: Coester, Carl-Fr., 59427 Unna (DE)
(72) Erfinder: Coester, Carl-Fr., 59427 Unna (DE)
(74) Vertreter: Fritz & Brandenburg Patentanwälte
(86) Internationale Anmeldenummer: PCT/EP2003/005300
(87) Internationale Veröffentlichungsnummer: WO 2003/099265

(56) Entgegenhaltungen:
- WO-A-00/61129
- WO-A-01/51469
- DE-A- 19 934 432
- DE-A- 19 939 756
- OKUN MICHAEL S ET AL: "Depression associated with Parkinson's disease: Clinical features and treatment." NEUROLOGY, Bd. 58, Nr. 4 Supplement 1, 26. Februar 2002 (2002-02-26), Seiten S63-S70, XP008020688 February 26, 2002 ISSN: 0028-3878
- JORG J: "Treatment schedule for Parkinson's disease" DEUTSCHE MEDIZINISCHE WOCHENSCHRIFT 1983 GERMANY, Bd. 108, Nr. 28-29, 1983, Seiten 1116-1122, XP008020684
- PASSERO S ET AL: "Depressive symptomatology in Parkinson's disease: Treatment with trazodone" RASSEGNA DI STUDI PSICHIATRICI 1983 ITALY, Bd. 72, Nr. 1, 1983, Seiten 86-93, XP008020679
- ANANTH J: "Choosing the right antidepressant" PSYCHIATRIC JOURNAL OF THE UNIVERSITY OF OTTAWA 1983 CANADA, Bd. 8, Nr. 1, 1983, Seiten 20-26, XP008020694
- MAYNES D ET AL: "Treatment of depression in Parkinson's disease" NOVA SCOTIA MEDICAL BULLETIN 1986 CANADA, Bd. 65, Nr. 1, 1986, Seiten 14-17, XP008020682
- BENAZZI F: "Parkinson's disease worsened by nefazodone." INTERNATIONAL JOURNAL OF GERIATRIC PSYCHIATRY. ENGLAND DEC 1997, Bd. 12, Nr. 12, Dezember 1997 (1997-12), Seite 1195 XP008020678 ISSN: 0885-6230
- ALBANESE A ET AL: "Can trazodone induce parkinsonism?" CLINICAL NEUROPHARMACOLOGY. UNITED STATES APR 1988, Bd. 11, Nr. 2, April 1988 (1988-04), Seiten 180-182, XP008020687 ISSN: 0362-5664
- GENGO F M ET AL: "THE RELATIVE ANTIHISTAMINIC AND PSYCHOMOTOR EFFECTS OF HYDROXYZINE AND CETIRIZINE" CLINICAL PHARMACOLOGY AND THERAPEUTICS, Bd. 42, Nr. 3, 1987, Seiten 264-272, XP008024867 ISSN: 0009-9236
- GENGO F M ET AL: "ANTIHISTAMINES DROWSINESS AND PSYCHOMOTOR IMPAIRMENT CENTRAL NERVOUS SYSTEM EFFECT OF CETIRIZINE" ANNALS OF ALLERGY, Bd. 59, Nr. 6 PART 2, 1987, Seiten 53-57, XP008024866 SYMPOSIUM ON CETIRIZINE, A RECENT ADVANCE IN SELECTIVE ANTIHISTAMINE THERAPY, NAPLES, FLORIDA, USA, ISSN: 0003-4738
- POIRIER J ET AL: "Debrisoquine metabolism in parkinsonian patients treated with antihistamine drugs" LANCET 1987 UNITED KINGDOM, Bd. 2, Nr. 8555, 1987, Seite 386 XP001160877
- PEGGS J F ET AL: "Antihistamines: the old and the new." AMERICAN FAMILY PHYSICIAN. UNITED STATES AUG 1995, Bd. 52, Nr. 2, August 1995 (1995-08), Seiten 593-600, XP000980218 ISSN: 0002-838X
- COHEN M M ET AL: "Pharmacotherapy of Parkinson's disease" JOURNAL OF THE MAINE MEDICAL ASSOCIATION 1977 UNITED STATES, Bd. 68, Nr. 4, 1977, Seiten 134-141+146, XP008024543
- WATANABE T ET AL: "RECENT ADVANCES IN NEUROPSYCHOPHARMACOLOGY OF THE CENTRAL HISTAMINERGIC NEURON SYSTEM" YAKUBUTSU, SEISHIN, KODO - JAPANESE JOURNAL OF PSYCHOPHARMACOLOGY, KAWASAKI, JP, Bd. 11, Nr. 2, 1. April 1991 (1991-04-01), Seiten 105-121, XP000673774 ISSN: 0285-5313

## Beschreibung

Bei der Parkinson'schen Krankheit kommt es zum Abbau von Nervenzellen im Gehirn, die für die Produktion des sogenannten Dopamins benötigt werden. Dopamin gehört zu den Botenstoffen im Gehirn, die den Informationsaustausch zwischen benachbarten Nervenzellen ermöglichen. Durch den Rückgang von Dopamin beim Parkinson Patienten überwiegen andere Botenstoffe, das heißt das Gleichgewichtsverhältnis der Botenstoffe ist gestört. Im Laufe der chronischen Erkrankung kommt es zu einem massiven Ungleichgewicht der Botenstoffkonzentrationen. Zu den Symptomen bei der Parkinson'schen Krankheit gehören insbesondere Koordinationsschwierigkeiten und Störungen der Beweglichkeit.

In den meisten Fällen beginnt die Krankheit erst im fortgeschrittenen Lebensalter, insbesondere etwa zwischen dem 60. und 70. Lebensjahr. In selteneren Fällen setzt der Krankheitsbeginn aber auch wesentlich früher ein, unter Umständen schon vor dem 30. Lebensjahr. Nach Schätzungen leiden weltweit mehr als eine Million Patienten an der Parkinson'schen Krankheit. Problematisch ist, dass oft die Krankheit im frühen Stadium nicht erkannt wird. Daher werden viele Patienten zunächst nicht behandelt.

Die vorliegende Erfindung betrifft die Verwendung von 2- [3- [4- (3-chlorphenyl)-1-piperazinyl]-propyl]- 1,2, 4-triazolo [4, 3-a] pyridin-3 (2-H) -on (Trazodon) oder eines pharmazeutisch verträglichen Salzes davon in Kombination mit 2- [3- [4- (3-chlorphenyl)-1-piperazinyl]-propyl]-5-ethyl-4-(2-phenoxyethyl)- 2H-1,2, 4-triazol-3 (4H) -on (Nefazodon) oder eines pharmazeutisch verträglichen Salzes davon zur Herstellung von Arzneimitteln zur Behandlung von Morbus Parkinson.

Eine der gängigen Behandlungsmethoden besteht darin, dass man den fehlenden Botenstoff Dopamin durch Zugabe entsprechender Arzneimittel ersetzt. Problematisch ist dabei, dass bei längerer Einnahme von L-Dopa dessen Wirksamkeit nachlässt, so dass die Dosierung erhöht werden muss. Hohe L-Dopa Dosierungen führen jedoch zu einer Reihe von Nebenwirkungen und bei längerer Einnahme zu unerwünschten Spätfolgen. Dies ist insbesondere bei denjenigen Patienten problematisch, bei denen die Krankheit bereits in vergleichsweise niedrigem Lebensalter ausbricht. Ein hoher Prozentsatz der mit L-Dopa behandelten Patienten zeigt bereits nach einigen Jahren motorische Störungen. Aus diesen Gründen ist man in letzter Zeit verstärkt dazu übergegangen, insbesondere zu Beginn der Therapie sogenannte Dopaminagonisten einzusetzen, die dann teilweise im fortgeschrittenen Stadium der Erkrankung mit L-Dopa kombiniert werden.

Aus dem Stand der Technik ist Nefazodon, ein Phenoxyethyltriazolinon-phenylpiperazin, als Antidepressivum bekannt geworden. Es wird angenommen, dass die antidepressive Wirkung von Nefazodon mit der Verstärkung der Serotonergenaktivität im zentralen Nervensystem zusammenhängt. Als Wirkstoff wird in den entsprechenden Arzneimitteln in der Regel das Nefazodonhydrochlorid verwendet. Dieses Mittel wird ausschließlich gegen depressive Erkrankungen eingesetzt. Vom Hersteller werden in der Gebrauchsinformation einige Nebenwirkungen angegeben, wobei als häufigere Nebenwirkungen betreffend das Nervensystem unter anderem angegeben wird, dass Störungen der Koordination von Bewegungsabläufen (Ataxie) und Verlangsamung von Bewegungsabläufen auftritt. Hieraus schließt der Fachmann, dass Nefazodon zur Behandlung der Parkinson'schen Krankheit, bei der ja unter anderem die vorgenannten Symptome auftreten, kontraindiziert ist. Der Fachmann hatte somit keine Veranlassung, die Wirksamkeit von Nefazodon bei der Behandlung von Morbus Parkinson zu prüfen. Über entsprechende Untersuchungen in dieser Richtung ist dem Anmelder folglich nichts bekannt.

Die exakte Formel für Nefazodon ist beispielsweise in der DE 34 43 820 C2 wiedergegeben und entspricht der nachfolgend angegebenen Strukturformel I

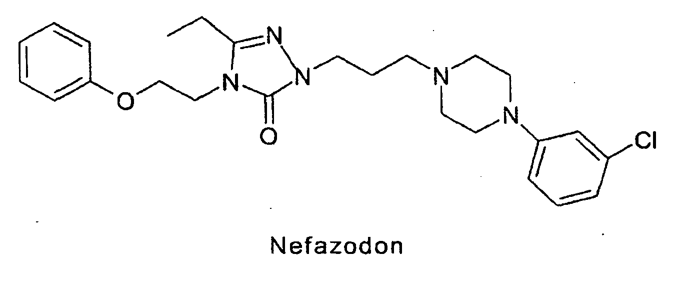

Die genaue Bezeichnung nach der chemischen Nomenklatur lautet 2-[3-[4-(3-Chlorphenyl)-1-piperazinyl]propyl]-5-ethyl-4-(2-phenoxyethyl)-2H-1,2,4-triazol-3(4H)-on. In der vorgenannten Druckschrift wird Nefazodon als antidepressiv wirksames Mittel bezeichnet. Weitere Indikationen werden nicht angegeben.

Michael S. Okun et al. untersuchen in einem Artikel mit der Überschrift "Depression associated with Parkinson's disease" in Neurology, Bd. 58, Nr.4 Supplement 1, 26. Februar 2002, Seiten S63-S70, verschiedene Antidepressiva im Hinblick auf deren mögliche Anwendung gegen Depressionen bei Parkinson-Patienten. Es wird auch die Wirksamkeit von Nefazodon bei der Behandlung von Depressionen bei Parkinson-Patienten beschrieben. Es wird jedoch nicht beschrieben, dass Nefazodon einen positiven Effekt auf die typischen sonstigen Symptome von Morbus Parkinson (außer der Depression selbst) zeigt.

In den Dokumenten DE 199 39 756 A, 199 34 432 A und WO 01/51469 A werden 5-HT 2A-Antagonisten verschiedener chemischer Struktur, u.a. Piperidine und Imidazolderivate und deren Verwendung zur Behandlung von Morbus Parkinson beschrieben. Die Substanzen Nefazodon und Trazodon werden jedoch in diesen Schriften nicht erwähnt.

Aufgrund der oben genannten Tatsache, dass der Dopaminverbrauch bei Parkinson Patienten nach längerer Behandlungszeit zunimmt, verbunden mit der Zunahme unerwünschter Nebenwirkungen und dem Auftreten von Langzeitschädigungen und angesichts der weiten Verbreitung der Krankheit, deren Häufigkeit zudem zuzunehmen scheint, besteht ein großes volkswirtschaftliches Bedürfnis daran, Medikamente zu finden, die eine Therapie ermöglichen, bei der die zu verabreichenden L-Dopamin-Dosen gesenkt werden können.

Die ältere nicht vorveröffentlichte Anmeldung DE 102 23 254.7, angemeldet am 24.05.2002, des Anmelders beschreibt bereits die

Verwendung der Triazolinonderivate 2-[3-[4-(3-chlorphenyl)-1-piperazinyl]-propyl]-5-ethyl-4-(2-phenoxyethyl)-2H-1,2,4-triazol-3(4H)-on (Nefazodon) beziehungsweise dessen pharmazeutisch verträgliche Salze und die Verwendung von 2-[3-[4-(3-chlorphenyl)-1-piperazinyl]-propyl]-1,2,4-triazolo[4,3-a]pyridin-3(2-H)-on (Trazodon) bei der Behandlung von Morbus Parkinson.

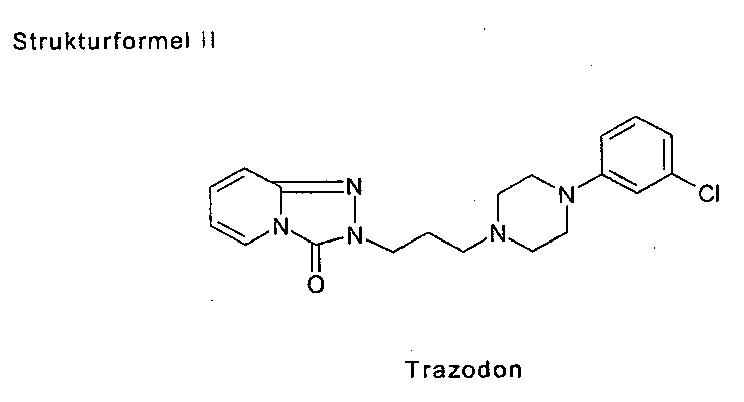

Überraschenderweise konnte festgestellt werden, dass diese bislang nur als Antidepressiva bekannten Substanzen eine außerordentlich gute therapeutische Wirkung bei der Behandlung der Krankheitssymptome von Morbus Parkinson zeigen. Im Anschluss an diese medizinisch und volkswirtschaftlich bedeutsamen Erkenntnisse wurden weitere klinische Studien mit Versuchsreihen an Patienten durchgeführt, durch die die erstaunliche Wirksamkeit der genannten Wirkstoffe bestätigt werden konnte. Dabei wurden aber zunächst nur jeweils entweder Nefazodon oder Trazodon als Wirkstoff für sich allein bei der Behandlung von Patienten eingesetzt. Bei dem Wirkstoff Trazodon hat sich als nachteilig erwiesen, dass dieser bei den Patienten nach der Einnahme eine gewisse Müdigkeit erzeugt. Die Einnahme dieses Wirkstoffs durch den Patienten am Tage ist daher weniger empfehlenswert.

Bei der Behandlung der Parkinson-Patienten mit den bislang üblichen Dopamin-Medikamenten war es bislang bei der Einnahme des Dopamin-Mittels am Abend üblich, zusätzlich ein sogenanntes Depotmittel dem den Wirkstoff Dopamin enthaltenden Medikament beizugeben, da anderenfalls eine unerwünschte zu rasche Freisetzung des Dopamins im Stoffwechsel des schlafenden Patienten erfolgte, so dass in nachteiliger Weise die Wirkung des Dopamins nicht über eine ausreichend lange Zeitdauer bis zum anderen Morgen hin anhielt. Die dabei üblicherweise verwendeten Depotmittel sind nach dem Stand der Technik sehr kostenaufwendig.

Die Aufgabe der vorliegenden Erfindung besteht folglich darin, ein Mittel zur Verfügung zu stellen, welches den Dopaminverbrauch beim Parkinson-Patienten senkt, eine gute Wirksamkeit zur Behandlung der Krankheitssymptome aufweist und dabei möglichst geringe bzw. nur unbedeutende Nebenwirkungen zeigt.

Die Lösung dieser Aufgabe liefert eine erfindungsgemäße Verwendung gemäß Anspruch 1 beziehungsweise eine pharmazeutische Wirkstoffkombination gemäß Anspruch 11.

Es hat sich bei Untersuchungen im Rahmen der vorliegenden Erfindung gezeigt, dass die Einnahme der vorgenannten erfindungsgemäßen Substanzen bei Parkinson-Patienten überraschenderweise zu einer erheblichen Senkung des DopaminVerbrauchs führt. In Patientenversuchen konnte gezeigt werden, dass Parkinson-Patienten, die regelmäßig L-Dopamin einnehmen, bei gleichzeitiger Einnahme von Nefazodon ihren Dopaminverbrauch gegenüber früher ganz wesentlich reduzieren können. Die bei zusätzlicher Einnahme von Nefazodon erforderliche Dopamin-Dosis kann in vielen Fällen auf beispielsweise die Hälfte oder sogar ein Drittel der bisher bei alleiniger Behandlung mit Dopamin erforderlichen Dosis gesenkt werden. Dabei wird erfindungsgemäß nicht nur die Tagesdosis des Dopamins gesenkt, sondern erstaunlicherweise bewirkt die Nefazodon-Einnahme auch eine bessere zeitliche Verteilung des eingenommenen Dopamins im Körper des Patienten. Dopamin hat gewöhnlich eine relativ kurze Halbwertzeit im menschlichen Körper, so dass seine Wirkung nicht sehr lange anhält. Bei der reinen Dopamintherapie muss der Parkinson-Patient das Dopamin daher relativ häufig über den Tag verteilt einnehmen, beispielsweise im Abstand von zwei bis zweieinhalb Stunden. Da die Wirkung des Dopamins zudem durch die gleichzeitige Aufnahme von Nahrungsmitteln beeinträchtigt wird, wird empfohlen eine zeitlang nach dessen Einnahme keine Nahrungsmittel zu sich zu nehmen. Dies führt zu einer wesentlichen Beeinträchtigung der Lebensqualität bei Parkinson-Patienten, insbesondere wenn die Krankheit bereits im fortgeschrittenen Stadium vorliegt und daher hohe Dosierungen des Dopamins und die relativ häufige Einnahme in kurzen zeitlichen Abständen erforderlich ist. Die erfindungsgemäße Verwendung insbesondere von Nefazodon bzw. dessen Derivaten führt dagegen in vorteilhafter Weise bei Patienten, die gleichzeitig Dopamin einnehmen zu einer zeitlich verteilten Wirkung des Dopamins. Offenbar entsteht aus bislang nicht bekannter Ursache eine Depot-Wirkung, bei der das Dopamin im Körper des Patienten aufgrund der Einnahme des Nefazodons langsamer freigesetzt wird. Dadurch kann die Dopamineinnahme nicht nur in geringeren Dosen sondern auch in größeren zeitlichen Abständen erfolgen.

Weiterhin wurde festgestellt, dass die erfindungsgemäße Verwendung von insbesondere Nefazodon und dessen Derivaten zu einer Senkung von Nebenwirkungen bei herkömmlichen Parkinson-Medikamenten führt, die Dopamin enthalten. Beispielsweise wird eine unangenehme Nebenwirkung des Dopamins, nämlich das Auftreten einer unkontrollierten Motorik beim Patienten, durch das Nefazodon positiv beeinflusst.

Ein weiterer Vorteil der erfindungsgemäßen Verwendung von Nefazodon liegt darin, dass dieses einfach oral, insbesondere in Tablettenform verabreicht werden kann, im Gegensatz zu anderen in letzter Zeit bekannt gewordenen Medikamenten gegen Parkinson, die gespritzt werden müssen und zum Teil nur vom Arzt gespritzt werden können, wenn die Spritze beispielsweise im Kopfbereich in unmittelbarer Nähe zur Hirnregion gesetzt werden muss.

Wenn der erfindungsgemäße Wirkstoff in Tablettenform verabreicht wird, enthält eine solche Tablette neben dem Wirkstoff selbst in der Regel übliche Hilfsstoffe. Beispielsweise kommen Hilfsstoffe in Betracht, wie sie für das im Handel befindliche Medikament "Nefadar ®" verwendet werden, insbesondere sind dies mikrokristalline Zellulose, Povidon, Poly(O-carboxymethyl)stärke, Natriumsalz, hochdisperses Siliciumdioxid, Magnesiumstearat, Eisenoxid oder dergleichen.

Weiterhin ist Nefazodon bzw. dessen übliche arzneimittelverträgliche Salze im allgemeinen gut verträglich und zeigt nur relativ wenig oder selten gravierende Nebenwirkungen.

Eine bevorzugte Dosierung im Rahmen der vorliegenden Erfindung liegt bei der täglichen Einnahme von einigen 100 mg, wobei diese Einnahme vorzugsweise über den Tag verteilt in mehreren Dosen, vorzugsweise durch Einnahme von Tabletten erfolgt. Üblicherweise enthalten die Tabletten den Wirkstoff in einer Menge von 100 mg oder 200 mg. Eine bevorzugte Tagesdosierung liegt beispielsweise im Bereich von etwa 300 bis 600 mg täglich, so dass diese durch Einnahme zwei bis drei mal täglich in Einzeldosen von 100 mg bzw. 200 mg verabreicht werden kann. Beispielsweise kann man wenn eine Gesamtdosis von täglich 500 mg vorgesehen ist morgens 200 mg, mittags 200 mg und abends 100 mg einnehmen. Die erfindungsgemäße Verabreichung des Nefazodon-Präparats führte dazu, dass der Dopaminverbrauch des Patienten erheblich gesenkt werden konnte. Beispielsweise konnte bei einem Patienten, bei dem die Krankheit bereits in vorgeschrittenem Stadium vorlag, die vor der erfindungsgemäßen Behandlung mit Nefazodon erforderliche Tagesdosis von 900 mg bis 1000 mg Dopamin täglich auf eine tägliche Gesamtdosis von nur 300 bis 400 mg, das heißt auf etwa ein Drittel gesenkt werden. Die Einnahme des L-Dopamins konnte in wesentlich geringeren Einzeldosen und gleichzeitig mit höherem zeitlichen Abstand erfolgen, beispielsweise in drei Einzeldosen von ca. 125 mg, die beispielsweise drei mal täglich, das heißt morgens, mittags und abends eingenommen wurden. Dies hatte für den Patienten den wesentlichen Vorteil, dass wegen des größeren zeitlichen Abstands der Einnahme des L-Dopamins die Einnahme der Mahlzeiten in einem gewöhnlichen Rhythmus wie bei einem gesunden Menschen ungestört erfolgen konnte.

Neben Nefazodon, kommt im Rahmen der erfindungsgemäßen Verwendung als Wirkstoff insbesondere ein anderes Triazolon in Betracht, welches ebenfalls als Substituenten eine Phenylpiperazin-Gruppe enthält, die über eine Propyl-Gruppe mit einem Stickstoffatom des Triazolonrings verbunden ist. Es handelt sich um das 1,2,4-triazolo[4,3-a]pyridin, welches unter dem Namen Trazodon bekannt geworden ist und in der US Patentschrift 4 338 317 als Antidepressivum beschrieben wird. Die Strukturformel II für Trazodon ist nachfolgend wiedergegeben

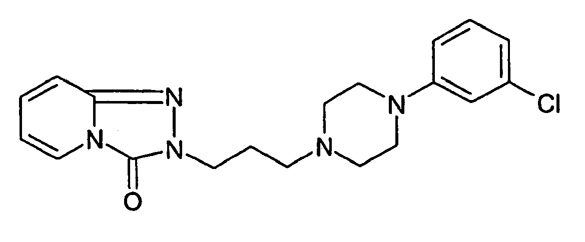

Die genaue Bezeichnung nach der chemischen Nomenklatur lautet 2-[3-[4-(3-chlorphenyl)-1-piperazinyl]-propyl]-1,2,4-triazolo[4,3-a]pyridin-3(2-H)-on.

Es ist zu vermuten, dass die sowohl in Trazodon als auch in dem oben genannten Nefazodon enthaltene Triazolinon-Gruppe bzw. die in beiden Fällen mehrere Übereinstimmungen aufweisenden Substituenten, nämlich die Propylphenylpiperazinyl-Gruppe einerseits bzw. die Substituenten in der 4 und in der 5 Postition des Triazolrings andererseits für die erfindungsgemäße Wirkung gegen die Parkinson'sche Krankheit verantwortlich sind. Der genaue Wirkmechanismus ist noch nicht erforscht.

Untersuchungen im Rahmen der vorliegenden Erfindung haben gezeigt, dass die zusätzliche Einnahme von Coffein, beispielsweise in Tablettenform die genannte positive Wirkung des Nefazodon unterstützt und zur einer weiteren Senkung des Dopaminverbrauchs beim Patienten führen kann. Hier wird beispielsweise die Einnahme einer Einzeldosis von etwa 50 mg bis etwa 0,2 g Coffein in Tablettenform empfohlen. Coffeintabletten mit dieser Wirkstoffdosis sind im Handel erhältlich. Weiterhin wurde festgestellt, dass auch die Einnahme von Acetylsalicylsäure die genannten positiven Wirkungen des Nefazodons unterstützen kann, so dass auch eine ergänzende Therapie mit Acetylsalicylsäure empfehlenswert sein kann. Es bietet sich beispielsweise die Einnahme von Acetylsalicylsäure in Tablettenform mit Einzeldosen von beispielsweise 500 mg pro Tablette an. Es sind auch Wirkstoffkombinationen von Coffein und Acetylsalicylsäure in einer Tablette möglich, wodurch sich der Vorteil ergibt, dass der Patient nur ein Medikament einnehmen muss.

Bei den zuvor beschriebenen weiteren Untersuchungen konnte überraschenderweise festgestellt werden, dass die genannte Tatsache, dass der Wirkstoff Trazodon beim Patienten eine gewisse Müdigkeit erzeugt in besonders vorteilhafter Weise therapeutisch genutzt werden kann. Das Arzneimittel mit dem Wirkstoff Trazodon wurde den Patienten abends verabreicht. Der Wirkstoff Trazodon wirkte beim Patienten schlaffördernd. Es wird davon ausgegangen, dass bei der Behandlung von Parkinson-Patienten mit Nefazodon oder auch Trazodon auch weiterhin eine ergänzende Einnahme von Dopamin durch den Patienten sinnvoll ist. Dies hängt sicher auch von dem jeweiligen Patienten und dem Stadium der Krankheit ab. Die Wirkstoffe Nefazodon und Trazodon sind daher mindestens in der Lage, eine Senkung der notwendigen Dosis des einzunehmenden Dopamins zu erreichen. Abends wurde den Patienten jedoch bislang aus den zuvor genannten Gründen das zu verabreichende Dopamin in Verbindung mit einem Depotmittel gegeben.

Durch weitere Untersuchungen konnte nun festgestellt werden, dass der Wirkstoff Trazodon selbst bei dem außerdem eingenommenen Dopamin-Medikament eine Depotwirkung erzeugt. Diese Erkenntnis führt zu dem entscheidenden Therapievorteil, das man nun bei abendlicher Einnahme von Trazodon das zusätzlich zu verabreichende Dopamin-Medikament ohne das bislang notwendige Depotmittel einnehmen kann, wobei dennoch die günstige langsame Freisetzung des Dopamins in der Schlafphase erfolgt. Da das Depotmittel entfällt führt dies zu einem erheblichen Kostenvorteil in der Therapie. Da außerdem das Trazodon schlaffördernd wirkt, wird darüber hinaus auch der Dopamin-Verbrauch in der Schlafphase gemindert. Am kommenden Morgen kann dann der Patient das für die Verabreichung tagsüber besser geeignete Nefazodon einnehmen. Bei den durchgeführten Untersuchungen stellte sich heraus, dass die Patienten nach dieser Nefazodon-Einnahme, bei der am Vorabend Trazodon eingenommen wurde, einen gelösten Eindruck machen. Die Kombination der Wirkstoffe Nefazodon und Trazodon in der Therapie von Morbus Parkinson führt also zu erstaunlich guten Ergebnissen. Dabei erbringt die kombinierte Therapie mit beiden Wirkstoffen, die gleichzeitig, getrennt oder zeitlich abgestuft eingenommen werden können in mehrerer Hinsicht Vorteile, die über die Wirkung bei einer Therapie mit jeweils nur einem der beiden Einzelwirkstoffe hinausgeht.

Besonders vorteilhaft und bevorzugt ist die zeitlich abgestufte Anwendung der beiden genannten Wirkstoffe in der Therapie von Morbus Parkinson. Ganz besonders vorteilhaft ist die Verwendung von Trazodon oder eines pharmazeutisch verträglichen Salzes davon oder eines dieses enthaltenden Mittels am Abend oder vor dem Schlafengehen in Kombination mit der Verwendung von Nefazodon oder eines pharmazeutisch verträglichen Salzes davon beziehungsweise eines dieses enthaltenden Mittels am Tage in einer oder mehreren Einzeldosen.

Besonders bevorzugt ist die Verwendung der genannten Wirkstoffe Nefazodon und/oder Trazodon in Arzneimitteln, die in Tablettenform vorliegen. Dabei wird als vorteilhaft angesehen, wenn eine für eine Einzeldosis vorgesehene Tablette zwischen etwa 50 mg und etwa 200 mg eines der Wirkstoffe Nefazodon oder Trazodon oder jeweils eine entsprechende Dosis beider Wirkstoffe enthält. Dabei kann es auch vorteilhaft sein, beide Wirkstoffe in einer Tablette zu kombinieren, wobei dann die Einzeldosis des einzelnen Wirkstoffs in der Tablette verringert werden kann, so dass beispielsweise auch eine Tablette jeweils nur 25 mg Nefazodon und 25 mg Trazodon enthalten könnte. Außerdem ist es möglich, das jeweilige Verhältnis der Wirkstoffanteile in einzelnen Tabletten oder gleichzeitig oder zeitlich versetzt einzunehmenden Kombinationspräparaten zu variieren, wobei es sich aus den zuvor genannten Gründen anbietet, die Dosierung des Wirkstoffs Nefazodon bei der Einnahme am Tage höher zu wählen als den jeweiligen Anteil an Trazodon und dieses Verhältnis zum Abend hin zu verschieben, so dass Abends einzunehmende Tabletten vorzugsweise überwiegend den Wirkstoff Trazodon und in einer nur geringeren Menge den Wirkstoff Nefazodon enthalten. Selbstverständlich ist es auch möglich, den Patienten Abends eine oder mehrere Tabletten einnehmen zu lassen, die nur Trazodon enthalten.

Der Wirkstoff Trazodon kann in dem diesen enthaltenden Arzneimittel als pharmazeutisch verträgliches Salz vorliegen, beispielsweise in Form des Hydrochlorids.

Parkinson-Patienten leiden häufig aufgrund ihrer Krankheit unter Depressionen. Aufgrund der Tatsache, dass bei nicht unter Parkinson leidenden depressiven Patienten die Einnahme von Nefazodon aber auch Trazodon zu Nebenwirkungen betreffend das Nervensystem führte, beispielsweise zu Störungen der Koordination von Bewegungsabläufen (Ataxie) und Verlangsamung von Bewegungsabläufen, ging man bislang davon aus, dass Nefazodon beziehungsweise Trazodon bei an Depressionen leidenden aber nicht von der Parkinson'schen Krankheit betroffenen Patienten kontraindiziert ist. Die nach der Feststellung der Wirksamkeit der Wirkstoffe Nefazodon und Trazodon bei der Behandlung der Symptome der Parkinson'schen Krankheit von dem Anmelder veranlassten Untersuchungen haben zu der Erkenntnis geführt, dass bei Parkinson-Patienten, insbesondere bei solchen, die parallel mit Dopamin-haltigen Medikamenten therapiert werden, die antidepressive Wirkung der Wirkstoffe Nefazodon beziehungsweise Trazodon besonders in Erscheinung tritt. Es ist daher nach den neuen Erkenntnissen, die sich aus den Untersuchungen im Zusammenhang mit der vorliegenden Erfindung ergeben, besonders empfehlenswert, die Wirkstoffe Nefazodon beziehungsweise Trazodon jeweils für sich genommen, in Kombination miteinander und/oder in Kombination mit Dopamin-haltigen Arzneimitteln spezifisch auch zur Behandlung von Depressionen bei Parkinson-Patienten einzusetzen.

Weitere Patientenstudien im Rahmen der vorliegenden Erfindung haben ergeben, dass bei manchen Patienten, die mit Nefazodon beziehungsweise Trazodon gegen Morbus Parkinson behandelt wurden zwar eine recht gute Wirkung im Hinblick auf einen Rückgang der Symptome beobachtet wurde, allerdings konnte bei einzelnen Patienten eine herausragende Wirkung beobachtet werden. Da es für dieses unterschiedlich gute Ansprechen auf die Wirkstoffe Nefazodon beziehungsweise Trazodon keine Erklärung gab, wurde dies zum Anlass genommen weitere Untersuchungen durchzuführen, um die Zusammenhänge aufzuklären.

Im Rahmen der genannten weiteren Studien konnte überraschenderweise festgestellt werden, dass die Einnahme eines Antihistaminikums eine gute Wirkung bei der Behandlung der Symptome der Parkinson'schen Krankheit zeigt. Eine besonders gute Wirkung zeigt das Antihistaminikum mit dem Wirkstoff Cetirizin beziehungsweise ein pharmazeutisch verträgliches Salz davon, insbesondere das Cetirizindihydrochlorid. Es handelt sich um ein relativ weit verbreitetes Antihistaminikum, welches zur Behandlung von Allergien wie beispielsweise Heuschnupfen, Juckreizbeschwerden und dergleichen eingesetzt wird. Cetirizin hat den Vorteil, dass keine relevanten Nebenwirkungen bekannt sind. Das Arzneimittel ist sehr gut verträglich und kann sogar von Kindern eingenommen werden. Es wirkt relativ rasch und es sind keine nennenswerten Wechselwirkungen mit anderen Arzneimitteln bekannt. In der Regel genügt eine einmalige tägliche Einnahme. Der Wirkstoff Cetirizin kann beispielsweise in Tablettenform eingenommen werden oder beispielsweise auch in Form einer Lösung oder Suspension, die oral zum Beispiel als Saft verabreicht wird. Das Arzneimittel ist in der Regel rezeptfrei erhältlich. Der Wirkstoff Cetirizin wirkt dadurch, dass er das Histamin im Körper blockiert.

## Patentansprüche

1. Verwendung von 2- [3- [4- (3-chlorphenyl)-1-piperazinyl]-propyl]- 1,2, 4-triazolo [4, 3-a] pyridin-3 (2-H) -on (Trazodon) oder eines pharmazeutisch verträglichen Salzes davon in Kombination mit 2- [3- [4- (3- chlorphenyl)-1-piperazinyl]-propyl]-5-ethyl-4-(2-phenoxyethyl)-2H-1,2, 4-triazol-3 (4H) -on (Nefazodon) oder eines pharmazeutisch verträglichen Salzes davon zur Herstellung von Arzneimitteln zur Behandlung von Morbus Parkinson.

2. Verwendung nach Anspruch 1, bei der das das Trazodon oder ein pharmazeutisch verträgliches Salz davon enthaltende Mittel für eine Einnahme am Abend vorgesehen ist.

3. Verwendung nach einem der Ansprüche 1 oder 2, bei der das Arzneimittel in Tablettenform vorliegt, wobei eine für eine Einzeldosis vorgesehene Tablette zwischen etwa 50 mg und etwa 200 mg eines der Wirkstoffe Nefazodon oder Trazodon oder jeweils eine entsprechende Dosis beider Wirkstoffe enthält.

4. Verwendung nach einem der Ansprüche 1 bis 3 zur Herstellung von Arzneimitteln zur Behandlung von Depressionen bei Parkinson-Patienten.

5. Verwendung nach einem der Ansprüche 1 bis 4 bei Patienten, die gleichzeitig mit L-Dopamin therapiert werden.

6. Verwendung nach einem der vorhergehenden Ansprüche, **dadurch gekennzeichnet, dass** dem Patienten eine Tagesdosis von zwischen etwa 300 und etwa 600 mg verabreicht wird.

7. Verwendung nach einem der vorhergehenden Ansprüche, **dadurch gekennzeichnet, dass** das Nefazodon bzw. dessen pharmazeutisch verträgliches Salz in zwei bis drei Einzeldosen verabreicht wird.

8. Verwendung nach einem der vorhergehenden Ansprüche, **dadurch gekennzeichnet, dass** das Nefazodon bzw. dessen pharmazeutisch verträgliches Salz in einer oder mehreren Einzeldosen von jeweils etwa 100 mg bis etwa 200 mg verabreicht wird.

9. Verwendung nach einem der vorhergehenden Ansprüche, **dadurch gekennzeichnet, dass** das Nefazodon oder sein pharmazeutisch verträgliches Salz in Tablettenform bestimmt zur oralen Einnahme verabreicht wird.

10. Verwendung nach einem der vorhergehenden Ansprüche in Verbindung mit der Einnahme eines Coffein und/oder Acetylsalicylsäure enthaltenden Mittels im gleichen Zeitraum zur Behandlung von Morbus Parkinson.

11. Pharmazeutische Wirkstoffkombination enthaltend 2- [3- [4- (3- chlorphenyl)-1-piperazinyl]-propyl]-5-ethyl-4- (2-phenoxyethyl)- 2H-1,2, 4-triazol-3 (4H) -on (Nefazodon) oder ein pharmazeutisch verträgliches Salz davon und 2- [3- [4- (3-chlorphenyl)-1- piperazinyl]-propyl]-1, 2, 4-triazolo [4,3-a] pyridin-3 (2-H)-on (Trazodon) als Kombinationspräparat zur gleichzeitigen, getrennten oder zeitlich abgestuften Anwendung in der Therapie von Morbus Parkinson.

12. Pharmazeutische Wirkstoffkombination nach Anspruch 11 enthaltend wenigstens ein Antihistaminikum oder ein pharmazeutisch verträgliches Salz davon neben Nefazodon oder Trazodon als Kombinationspräparat zur gleichzeitigen, getrennten oder zeitlich abgestuften Anwendung in der Therapie von Morbus Parkinson.

13. Pharmazeutische Wirkstoffkombination nach einem der Ansprüche 11 bis 12, **dadurch gekennzeichnet, dass** mindestens das Nefazodon/Trazodon in Tablettenform vorliegt, wobei eine für eine Einzeldosis vorgesehene Tablette zwischen etwa 50 mg und etwa 200 mg mindestens eines der Wirkstoffe Nefazodon und/oder Trazodon oder jeweils eine entsprechende Dosis beider Wirkstoffe enthält.

## Claims

1. Use of 2- [3- [4- (3-chlorphenyl)-1-piperazinyl]-propyl]- 1,2, 4-triazolo [4, 3-a] pyridin-3 (2-H) - one (Trazodone) or a pharmaceutically acceptable salt thereof in combination with 2- [3- [4-(3- chlorphenyl)-1-piperazinyl]-propyl]-5-ethyl-4-(2-phenoxyethyl)- 2H-1,2, 4-triazol-3 (4H) - one (Nefazodone) or a pharmaceutically acceptable salt thereof for the production of pharmaceuticals for the treatment of Morbus Parkinson.

2. Use according to claim 1, wherein the pharmaceutical comprising Trazodone or a pharmaceutically acceptable salt thereof is provided for a taking in the evening.

3. Use according to claim 1 or 2, wherein the pharmaceutical is provided in the form of tablets, whereby a tablet provided for a single dose contains between about 50 mg and about 200 mg of one of the active agents Nefazodone or Trazodone or a respective dose of both active agents.

4. Use according to one of claims 1 to 3 for the production of pharmaceuticals for the treatment of depressions of patients having Parkinson's disease.

5. Use according to one of claims 1 to 4 with patients, who are at the same time therapeutically treated with L-dopamine.

6. Use according to one of the foregoing claims, **characterized in that**, a daily dose rate of between about 300 mg and about 600 mg is administered to the patient.

7. Use according to one of the foregoing claims, **characterized in that** Nefazodone or its pharmaceutically acceptable salt is administered in two or three single dose rates.

8. Use according to one of the foregoing claims, **characterized in that** Nefazodone or its pharmaceutically acceptable salt is administered in one or several single dose rates of each about 100 mg to about 200 mg, respectively.

9. Use according to one of the foregoing claims, **characterized in that** Nefazodone or its pharmaceutically acceptable salt is administered in the form of tablets designed to be orally taken.

10. Use according to one of the foregoing claims in combination with the taking of a pharmaceutical comprising caffeine and/or acetylsalicylic acid in the same period for the treatment of Parkinson's disease.

11. Combination of pharmaceutically active agents comprising 2- [3- [4- (3- chlorphenyl)-1-piperazinyl]-propyl]-5-ethyl-4- (2-phenoxyethyl)- 2H-1,2, 4-triazol-3 (4H) -one (Nefazodone) or a pharmaceutically acceptable salt thereof and 2- [3- [4- (3-chlorphenyl)-1- piperazinyl]-propyl]-1, 2, 4-triazolo [4,3-a] pyridin-3 (2-H)-one (Trazodone) as a combined drug for simultaneous, separate or step-by-step (graduated) application in treatment of Parkinson's disease.

12. Combination of pharmaceutically active agents according to claim 11 comprising at least one antihistamine or a pharmaceutically acceptable salt thereof in addition to Nefazodone or Trazodone as a combined drug for simultaneous, separate or step-by-step (graduated) application in treatment of Parkinson's disease.

13. Combination of pharmaceutically active agents according to one of claims 11 to 12, **characterized in that** at least Nefazodone/Trazodone is provided in the form of tablets, whereby a tablet provided for a single dose rate contains between about 50 mg and about 200 mg of at least one of the pharmaceutically active agents Nefazodone and/or Trazodone or a corresponding dose rate of both active agents, respectively.

## Revendications

1. Utilisation de la 2-[3-[4-(3-chlorophényl)-1-pipérazinyl]-propyl]-1,2,4-triazolo[4,3-a]pyridin-3(2-H)-one (trazodone) ou d'un sel pharmaceutiquement acceptable de celle-ci en combinaison avec la 2-[3-[4-(3-chlorophényl)-1-pipérazinyl]-propyl]-5-éthyl-4-(2-phénoxyéthyl)-2H-1,2,4-triazolo-3(4H)-one (néfazodone) ou un sel pharmaceutiquement acceptable de celle-ci, pour la fabrication de médicaments destinés au traitement de la maladie de Parkinson.

2. Utilisation selon la revendication 1, dans laquelle la substance contenant la trazodone ou un sel pharmaceutiquement acceptable de celle-ci est prévue pour être prise le soir.

3. Utilisation selon l'une quelconque des revendications 1 ou 2, dans laquelle le médicament se présente sous forme de comprimé, un comprimé prévu pour une dose unique contenant entre 50 mg à peu près et 200 mg à peu près de l'une des substances actives, à savoir de la néfazodone ou de la trazodone, ou contenant respectivement une dose correspondante des deux substances actives.

4. Utilisation selon l'une quelconque des revendications 1 à 3, pour la fabrication de médicaments destinés au traitement de dépressions chez les patients souffrant de la maladie de Parkinson.

5. Utilisation selon l'une quelconque des revendications 1 à 4, chez les patients qui sont traités en même temps avec la L-dopamine.

6. Utilisation selon l'une quelconque des revendications précédentes, **caractérisée par le fait que** le patient reçoit une dose quotidienne comprise entre environ 300 et environ 600 mg.

7. Utilisation selon l'une quelconque des revendications précédentes, **caractérisée par le fait que** la néfazodone ou bien son sel pharmaceutiquement acceptable est administré(e) en deux à trois doses uniques.

8. Utilisation selon l'une quelconque des revendications précédentes, **caractérisée par le fait que** la néfazodone ou bien son sel pharmaceutiquement acceptable est administré(e) en une ou plusieurs dose(s) unique(s) comprise(s) chacune entre 100 mg à peu près et 200 mg à peu près.

9. Utilisation selon l'une quelconque des revendications précédentes, **caractérisée par le fait que** la néfazodone ou son sel pharmaceutiquement acceptable est administré(e) sous forme de comprimés destinés à une administration par voie orale.

10. Utilisation selon l'une quelconque des revendications précédentes, en relation avec l'administration d'une substance contenant de la caféine et/ou de l'acide acétylsalicylique, durant la même période, pour le traitement de la maladie de Parkinson.

11. Combinaison de substances actives pharmaceutiques comprenant la 2-[3-[4-(3-chlorophényl)-1-pipérazinyl]-propyl]-5-éthyl-4-(2-phénoxyéthyl)-2H-1,2,4-triazolo-3(4H)-one (néfazodone) ou un sel pharmaceutiquement acceptable de celle-ci ainsi que la 2-[3-[4-(3-chlorophényl)-1-pipérazinyl]-propyl]-1,2,4-triazolo[4,3-a]pyridin-3(2-H)-one (trazodone), en tant que préparation combinée pour l'application simultanée, séparée ou échelonnée dans le temps, dans la thérapie de la maladie de Parkinson.

12. Combinaison de substances actives pharmaceutiques selon la revendication 11, comprenant au moins un antihistaminique ou un sel pharmaceutiquement acceptable de celui-ci, outre la néfazodone ou la trazodone, en tant que préparation combinée pour l'application simultanée, séparée ou échelonnée dans le temps, dans la thérapie de la maladie de Parkinson.

13. Combinaison de substances actives pharmaceutiques selon l'une quelconque des revendications 11 à 12, **caractérisée par le fait qu'**au moins la néfazodone/trazodone est présente sous forme de comprimé, un comprimé prévu pour une dose unique contenant entre 50 mg à peu près et 200 mg à peu près de l'une au moins des substances actives, à savoir de la néfazodone et/ou de la trazodone, ou contenant respectivement une dose correspondante des deux substances actives.
